# EUROPEAN PATENT SPECIFICATION

(11) **EP 2 860 173 B1**
(45) Date of publication and mention of the grant of the patent: **05.05.2021**
(21) Application number: 13804801.2
(22) Date of filing: 07.06.2013
(51) Int. Cl.: C07C 247/04, C07D 257/04

(54) **METHOD FOR PRODUCING AZIDE COMPOUND, AND METHOD FOR PRODUCING 1H-TETRAZOLE DERIVATIVE**
VERFAHREN ZUR HERSTELLUNG EINER AZIDVERBINDUNG UND VERFAHREN ZUR HERSTELLUNG EINES 1H-TETRAZOLDERIVATS
PROCÉDÉ DE FABRICATION D'UN COMPOSÉ AZIDE ET PROCÉDÉ DE FABRICATION DE DÉRIVÉ DE 1H-TÉTRAZOLE

(30) Priority: 12.06.2012 JP 2012132812
(43) Date of publication of application: 15.04.2015
(73) Proprietor: Nippon Soda Co., Ltd., Tokyo 100-8165 (JP)
(72) Inventor: MIYAZAKI Hidekazu, Takaoka-shi Toyama 933-8507 (JP)
(74) Representative: Mewburn Ellis LLP
(86) International application number: PCT/JP2013/065833
(87) International publication number: WO 2013/187325

(56) References cited:
- WO-A1-2010/103783
- WO-A1-2011/110651
- M. VEERA NARAYANA REDDY ET AL: "Synthesis, antioxidant and antimicrobial activity of novel benzene-1,4-diamine-bis-dioxaphosphepine-6 [lambda] 5 iminophosphoranes", JOURNAL OF HETEROCYCLIC CHEMISTRY, 1 January 2010 (2010-01-01), pages NA-NA, XP055222225, US ISSN: 0022-152X, DOI: 10.1002/jhet.358
- HANS BOCK ET AL: "Analyse und Optimierung von Gasphasen-Reaktionen, 181,2) Die Pyrolyse von Methylazid", CHEMISCHE BERICHTE, vol. 114, no. 1, 1 January 1981 (1981-01-01), pages 220-226, XP055222229, DE ISSN: 0009-2940, DOI: 10.1002/cber.19811140123
- SCRIVEN E F V ET AL: "Azides: Their Preparation and Synthetic Uses", CHEMICAL REVIEWS, AMERICAN CHEMICAL SOCIETY, US, vol. 88, no. 2, 1 January 1988 (1988-01-01), pages 297-368, XP002525610, ISSN: 0009-2665, DOI: 10.1021/CR00084A001
- BERNHARD GUTMANN ET AL: "Synthesis of 5-Substituted 1H-Tetrazoles from Nitriles and Hydrazoic Acid by Using a Safe and Scalable High-Temperature Microreactor Approach", ANGEWANDTE CHEMIE INTERNATIONAL EDITION, vol. 49, no. 39, 18 August 2010 (2010-08-18), pages 7101-7105, XP55176761, ISSN: 1433-7851, DOI: 10.1002/anie.201003733
- PRAKASH B. PALDE ET AL: "Safe and Efficient Tetrazole Synthesis in a Continuous-Flow Microreactor", ANGEWANDTE CHEMIE INTERNATIONAL EDITION, vol. 50, no. 15, 4 April 2011 (2011-04-04) , pages 3525-3528, XP055009296, ISSN: 1433-7851, DOI: 10.1002/anie.201006272
- GUTMANN, B. ET AL.: 'Synthesis of 5-Substituted 1H-Tetrazoles from Nitriles and Hydrazoic Acid by Using a Safe and Scalable High-Temperature Microreactor Approach' ANGEW. CHEM. INT. ED. vol. 49, 2010, pages 7101 - 7105, XP055176761
- PALDE, P. B. ET AL.: 'Safe and Efficient Tetrazole Synthesis in a Continuous-Flow Microreactor' ANGEW. CHEM. INT. ED. vol. 50, 2011, pages 3525 - 3528, XP055009296
- SHIN'ICHIRO FUSE: 'Organic Synthesis Using Microflow Reactor' JOURNAL OF SYNTHETIC ORGANIC CHEMISTRY 0037-9980 vol. 70, no. 2, February 2012, JAPAN, pages 177 - 178, XP008175525
- Bernhard Gutmann ET AL: "Safe Generation and Synthetic Utilization of Hydrazoic Acid in a Continuous Flow Reactor", JOURNAL OF FLOW CHEMISTRY, vol. 2, no. 1, 21 March 2012 (2012-03-21), pages 8-19, XP55541905, HU ISSN: 2062-249X, DOI: 10.1556/jfchem.2012.00021
- JOHAN C BRANDT ET AL: "Controlling hazardous chemicals in microreactors: Synthesis with iodine azide", BEILSTEIN JOURNAL OF ORGANIC CHEMISTRY, vol. 5, 1 January 2009 (2009-01-01), XP055675630, DOI: 10.3762/bjoc.5.30
- HANS. BOCK ET AL: "Gas-phase reactions. 66. Gas-phase pyrolyses of alkyl azides: experimental evidence for chemical activation", JOURNAL OF THE AMERICAN CHEMICAL SOCIETY, vol. 110, no. 16, 1 August 1988 (1988-08-01), pages 5261-5269, XP055675615, US ISSN: 0002-7863, DOI: 10.1021/ja00224a004

## Description

### TECHNICAL FIELD

The present invention relates to a method for producing an azide compound and a method for producing a 1H-tetrazole derivative. More particularly, the present invention relates to a method for synthesizing an azide compound by reacting in a flow reactor an azide of an alkaline metal or alkaline earth metal that has low risk of explosion and the like as rawmaterial, and a method for producing a 1H-tetrazole derivative from the resulting azide compound.

The present application claims priority on the basis of Japanese Patent Application No. 2012-132812 filed in Japan on June 12, 2012.

### BACKGROUND ART

Numerous types of control agents have been proposed for use against diseases of agricultural and horticultural crops. For example, Patent Document 1 discloses a tetrazoyl oxime derivative having superior pharmacological efficacy for useful plants, and proposes the use thereof as a plant disease control agent. Examples of methods for producing the tetrazoyl oxime derivative disclosed in Patent Document 1 include the method described in Patent Document 2 whereby a hydroxylamine is reacted with a 1-alkyl-5-benzoyl-1H-tetrazole derivative represented by the following general formula (P) and the resulting tetrazoylhydroxyimino derivative is used as raw material to produce a tetrazoyl oxime derivative. In general formula (P), A' represents a halogen atom, alkyl group, alkoxy group, methanesulfonyl group, trifluoromethyl group, aryl group, cyano group or nitro group, n represents an integer of 0 to 5, and Y' represents an optionally substituted alkyl group.

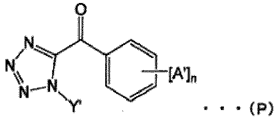

The method for producing the 1-alkyl-5-benzoyl-1H-tetrazole derivative represented by general formula (P) preferably consists of directly reacting benzoyl cyanide and an alkyl azide followed by forming a tetrazole ring by a cycloaddition reaction since it is easy to control the locations of substituents on the tetrazole ring.

An alkyl azide used as raw material of the aforementioned reaction is typically synthesized by alkylating sodium azide with an alkylating agent. For example, Non-Patent Document 1 reports that methyl azide was synthesized by methylating sodium azide with a methylating agent. Here, the alkyl azide is frequently a compound that is a highly explosive, highly dangerous compound in the manner of methyl azide and the like. Consequently, a method is sought that enables alkyl azide to be produced more safely and efficiently using comparatively safe sodium azide as raw material.

In addition, Patent Document 3 reports that a compound represented by general formula (P') (wherein, A' and n are the same as in general formula (P)) was able to be synthesized efficiently and safely by reacting an alkyl isocyanide such as methyl isocyanide with an acid halide such as benzoyl chloride followed by reacting the aforementioned reaction product with sodium azide. However, the method described in the aforementioned document requires that the methyl isocyanide be isolated due to concerns over explosion and toxicity. Moreover, it is extremely difficult to industrialize the aforementioned method due to the extremely distressing odor of methyl isocyanide.

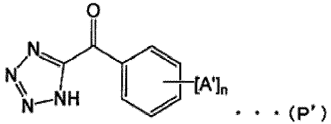

In addition, Non-Patent Document 2 reports that a tetrazole cyclization reaction was carried out by reacting benzyl cyanide and sodium azide in a flow reactor to a generate hydrogen azide a little at a time followed by immediately reacting with a nitrile. However, a reaction using benzoyl cyanide and methyl azide as raw materials is not disclosed in the aforementioned document.

### Prior Art Documents

### Patent Documents

Patent Document 1: International Publication No. WO 2003/016303
Patent Document 2: International Publication No. WO 2010/103783
Patent Document 3: International Publication No. WO 2011/110651

### Non-Patent Documents

Non-Patent Document 1: Dimroth, O. and Wislicenus, W., Chemische Berichte, 1905, Vol. 38, p. 1573
Non-Patent Document 2: Gutmann, et al., Angewandte Chemie International Edition, 2010, Vol. 49(39), p. 7101-7105 M. VEERA NARAYANA REDDY et al: "Synthesis, antioxidant and antimicrobial activity of novel benzene-1,4-diamine-bisdioxaphosphepine-6[1ambda] 5 iminophosphoranes", JOURNAL OF HETEROCYCLIC CHEMISTRY, 1 January 2010, pp538-542 describes a class of benzene-1,4-diamine-bis-dioxaphosphepine-6λ iminophosphoranes which were synthesized by the reaction of 6-chlorodibenzo[*d*,*f*] [1,3,2]dioxaphosphepine with 1,4-diaminobenzene to form bis-dibenzo[*d*,*f*][1,3,2]dioxaphosphepin-6-yl-benzene-diamine. Its subsequent reaction with different alkyl/aryl azides in tetrahydrofuran at 50-60°C under inert atmosphere yielded the title compounds.

HANS BOCK et al.: "Analyse und Optimierung van GasphasenReaktionen, 181,2) Die Pyrolyse van Methylazid", CHEMISCHE BERICHTE, vol. 114, no. 1, 1 January 1981, pages 220-226, describes that the PE spectroscopically analyzed pyrolysis of methyl azide occurs at 850 K and yields as main product methanimine, which according to a MNDO singlet hypersurface should be considerably stabilized relative to the tautomeric methyl nitrene.

SCRIVEN E F V et al.: "Azides: Their Preparation and Synthetic Uses", CHEMICAL REVIEWS, AMERICAN CHEMICAL SOCIETY, US, vol. 88, no. 2, 1 January 1988, pages 297-368, describes a wide variety of azides, their preparation, their reactions and their uses.

### DISCLOSURE OF THE INVENTION

### Problems to be Solved by the Invention

An object of the present invention is to provide a method for efficiently and safely producing an azide compound in a flow reactor using a comparatively safe azide of an alkaline metal or alkaline earth metal as raw material. Also described herein is a method for producing a 1H-tetrazole derivative from the resulting azide compound.

### Means for Solving the Problems

As a result of conducting extensive studies to solve the aforementioned problems, the inventor of the present invention found that an azide compound can be industrially safely and efficiently produced using sodium azide as raw material by using a flow reactor for the reaction vessel, thereby leading to completion of the present invention.

Namely, the method for producing an azide compound of the present invention include the aspects described in [1] and [2] below.
[1] A method for producing an azide compound, including: reacting an alkylating agent with sodium azide in a flow reactor to produce an azide compound represented by the following general formula (I):

   **Y-N₃ ···** **(I)**

   wherein, Y represents an alkyl group having 1 to 3 carbon atoms, in the state of a solution; and
   recovering the azide compound represented by general formula (I) in the state of a solution,
   wherein the reaction is carried out at a reaction temperature ranging from 35°C to 80°C, and the boiling point of the azide compound produced is lower than the reaction temperature,
   wherein a solution containing the alkylating agent and a solution containing the sodium azide are respectively and separately introduced into the flow reactor, and
   the solvent of the solution containing the sodium azide contains water, and the solvent of the solution containing the alkylating agent is an organic solvent capable of dissolving the azide compound represented by the general formula (I) and capable of undergoing phase separation with water, wherein the organic solvent is toluene
   and the alkylating agent is a compound represented by formula (III-B): 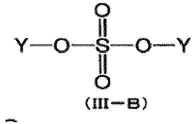 wherein, Y is the same as defined in the general formula (I) .
[2] The method for producing an azide compound described in [1] above, wherein the solution containing the sodium azide further contains a base.

### Effects of the Invention

The method for producing an azide compound of the present invention enables an azide compound to be produced industrially safely in the state of a solution (namely, in the state of being dissolved in a solvent) by using a flow reactor.

In addition, described herein is a method for producing a 1H-tetrazole derivative which method enables a 1H-tetrazole derivative to be produced by directly reacting an azide compound produced according to the method for producing an azide compound of the present invention with a cyanide compound without having to remove outside the flow reactor.

Namely, the method for producing a 1H-tetrazole derivative is characterized by carrying out a stepwise reaction in a flow reactor using an azide of an alkaline metal or alkaline earth metal as raw material that has low risk of explosion or other danger. As a result, a 1H-tetrazole derivative, which has substituents at the 1-position and 5-position and is useful as a synthesis raw material of active ingredients of agricultural chemicals and various other types of chemicals, can be produced industrially safely, in a short period of time and with high efficiency.

### BEST MODE FOR CARRYING OUT THE INVENTION

Although the following provides an explanation of preferred embodiments of the present invention, the present invention is not limited to these embodiments. Constituents of the present invention can be added, omitted, substituted or altered in other ways within a range that does not deviate from the gist of the present invention.

### <Method for Producing Azide Compound>

The method for producing an azide compound of the present invention is characterized by reacting an alkylating agent of formula (III-B) with sodium azide in a flow reactor to produce an azide compound represented by the following general formula (I) in the state of a solution. In other words, the azide compound produced according to the method for producing an azide compound of the present invention can be recovered as a solution.

**Y-N₃ ···** **(I)**

In general formula (I), Y represents a linear alkyl group having 1 to 2 carbon atoms.

There are some azide compounds in the manner of methyl azide that have a boiling point that is lower than the reaction temperature between an azide and alkylating agent and the like. Therefore, azide compounds in the gaseous state are removed from the reaction system by a procedure such as distillation. As a result, since the azide compound is recovered in the state of liquid having an extremely high concentration, there is a high risk of explosion and the like. In contrast, in the method for producing an azide compound of the present invention, since a flow reactor is used for the reaction vessel, the reaction can be carried out at a suitable pressure and the synthesized azide compound can be recovered in a state of being dissolved in a solvent.

### [Alkylating Agent]

In the method for producing an azide compound of the present invention, the sodium ion of sodium azide is substituted with a linear alkyl group having 1 to 2 carbon atoms by an alkylating agent. As a result, the azide compound represented by general formula (I) can be synthesized efficiently.

The alkylating agent used in the present invention is a compound represented by the following formula (III-B). Furthermore, these compounds can be synthesized from known compounds using known chemical reactions. 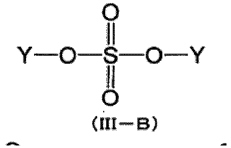 In general formulas (III-B) , Y is the same as defined in general formula (I).

Specific examples of the linear alkyl group are a methyl group, and an ethyl group.

The compound represented by general formulas (III-B) is that in which Y represents a linear alkyl group having 1 to 2 carbon atoms. Among these, the compound represented by general formula (III-B) is particularly preferably that in which Y represents a methyl group, namely a dimethyl sulfate. In the method for producing an azide compound of the present invention, since the reaction is carried out in a flow reactor, an azide compound can be safely and efficiently produced in the state of a solution even in the case the synthesized product is a lower alkyl azide having 6 or fewer carbon atoms that is generally considered to be explosive and toxic due to the low boiling point thereof.

### [Flow Reactor]

In the method for producing an azide compound of the present invention, a flow reactor is used for the reaction vessel used to react sodium azide (to also be simply referred to as an "azide") and the substituent Y introducing agent (the alkylating agent of formula (III-B)). Consequently, the reaction can be carried out comparatively safely in a low temperature environment even in a reaction in which a highly decomposable compound in the manner of an azide compound is obtained. Moreover, the synthesized azide compound can be recovered in the state of a solution by carrying out the reaction at a pressure of atmospheric pressure to 10 MPa.

The flow reactor used in the method for producing an azide compound of the present invention is provided with raw material introduction ports, a product discharge port and a flow path communicating there between. Raw materials are supplied through the aforementioned raw material introduction ports and the product obtained by a reaction that occurs in the aforementioned flow path is removed from the aforementioned product discharge port. The aforementioned flow path may also be provided with at least one of an introduction path, mixer portion and reactor portion (retention portion) as necessary. In the case the flow reactor is provided with a mixer portion, the portion of the flow path communicating between the raw material introduction ports and the mixer portion is referred to as an introduction path, while in the case a mixer portion is not present, the portion of the flow path that communicates between the raw material introduction ports and the reactor portion is referred to as an introduction path. In addition, the raw material introduction ports are normally connected to containers filled with raw materials. The product discharge portion may also be connected to a container for storing the product as necessary.

The mixer portion is a site that has the function of mixing a plurality of liquids by dispersion, and solutions supplied from the plurality of raw material introduction ports merge in the mixer portion. In addition, the reactor portion is a site where a reaction is carried out for synthesizing a product (azide compound represented by general formula (I) in the method for producing an azide compound of the present invention) from a plurality of raw material compounds (azide and substituent Y introducing agent in the method for producing an azide compound of the present invention). In the case of being provided with both a mixer portion and a reactor portion, the mixer portion is provided on the side of the raw material introduction ports. In the case of preliminarily supplying a reaction solution obtained by mixing all of the raw material compounds from a single raw material introduction port, the mixer portion need not be provided. In addition, the mixer portion may also serve as a reactor portion and the reactor portion need not be provided in the case the time required by the reaction for synthesizing the product is short and the reaction is able to be completed by the time the raw materials pass through the mixer portion.

In the case the flow reactor is provided with a plurality of raw material introduction ports and introduction paths, the upstream side of the flow path of the aforementioned flow reactor has a configuration in which it is branched corresponding to the number of introduction paths, and is further provided with at least one mixer portion. There are no particular limitations on the number of raw material introduction ports and introduction paths, and can be suitably selected corresponding the particular purpose. In the case of having three or more raw material introduction ports and introduction paths, a configuration may be employed in which liquid supplied from all of the introduction ports merges in a single mixer portion, or a configuration may be employed in which the liquid is allowed to merge in a stepwise manner in two or more mixer portions. For example, after merging liquid supplied from two introduction paths in a first mixer portion, the mixture discharged from the aforementioned mixer portion and liquid introduced from the remaining introduction path can be merged in a second mixer portion.

Furthermore, a portion of the raw materials can be preliminarily charged into the flow path of the flow reactor (such as the mixer portion), and the remaining raw materials may be respectively supplied from one or a plurality of raw material introduction ports.

There are no particular limitations on the material of the aforementioned flow reactor, and can be suitably selected corresponding to the required performance, such as heat resistance, pressure resistance, solvent resistance or processing ease. Examples of the aforementioned material include stainless steel, titanium, copper, nickel, aluminum, silicon, fluororesins such as Teflon® or perfluoroalkoxy resin (PFA), trifluoroacetoamide (TFAA) and polyether ether ketone resin (PEEK).

In addition, the material may be substantially the same throughout the entire flow path or different materials may be used for the introduction path, mixer portion and reactor portion, respectively.

There are no particular limitations on the cross-sectional shape of the flow path and may be rectangular, including a square or oblong shape, polygonal, including a triangular or pentagonal shape, star-shaped, or circular, including semicircular or elliptical shape. The cross-sectional shape of the flow path is not required to be constant. Furthermore, "flow path cross-section" refers to the cross-section in a direction perpendicular to the direction of flow of a reaction solution and the like through the flow path, while "cross-sectional area" refers to the area of the aforementioned cross-section.

There are no particular limitations on the cross-sectional area or flow path length of the flow path, and are suitably adjusted in consideration of such factors as viscosity and flow rate of the reaction solution, reaction temperature or reaction time. If the cross-sectional area of the flow path is excessively small, pressure loss becomes high thereby making it difficult to supply raw materials and allow the reaction solution to flow. Conversely, if the cross-sectional area is excessively large, heat exchange efficiency decreases and a temperature distribution and the like occur, thereby reducing the characteristics of the flow reactor. The cross-sectional area of the flow path may be substantially the same throughout the entire flow path or the cross-sectional area may be different in the introduction path, mixer portion and reactor portion, respectively. In the case the aforementioned flow reactor has a plurality of introduction paths, the cross-sectional area of each introduction path may be mutually the same or different.

The mixer portion has a function that mixes a plurality of liquids by dispersion and a function that removes the heat of reaction.

There are no particular limitations on the type of mixing used to mix liquids in the mixer portion, and can be suitably selected corresponding to the particular purpose. For example, mixing may be carried out by laminar flow or turbulent flow.

There are no particular limitations on the mixer portion provided it is provided with a structure that enables mixing of a plurality of liquids, and can be suitably selected corresponding to the particular purpose. Examples of the mixer portion include a T-pipe, micromixer and branched pipe. A T-shape or a Y-shape can be used for the shape of the mixer portion in the case the number of introduction ports is two, while a cross-shape, for example, can be used in the case the number of introduction ports is three.

There are no particular limitations on the cross-sectional area of the mixer portion provided it does not impair the effects of the present invention, and can be suitably adjusted in consideration of such factors such as the type of mixing. Since the mixer portion is able to favorably demonstrate both of the functions of mixing a plurality of liquids by dispersion and removing the heat of reaction, in the case the cross-sectional shape is circular, the mixer portion preferably has an inner diameter of about 10 µm to about 5 cm. In addition, although the cross-sectional area of the mixer portion may be the same as other portions such as the introduction paths, it is preferably larger than the introduction paths from the viewpoint of mixing efficiency.

There are no particular limitations on the flow path length of the mixer portion, and can be suitably adjusted in consideration of such factors as the type of mixing, type and amount of liquid supplied from each introduction path, or the presence or absence of a reactor portion. For example, in the case the cross-sectional shape is circular, the inner diameter can be about 10 µm to about 5 cm and the flow path length can be 10 cm to 50 m.

Although the flow path length of the mixer portion is preferably of a sufficient length for mixing liquids introduced from a plurality of introduction paths by dispersion, in the case of providing a separate reactor portion, the flow path length may be shorter. On the other hand, in the case of not providing a separate reactor portion and obtaining a product following completion of the reaction at the point the liquids have passed through the mixer portion, the flow path length of the mixer portion is preferably suitably adjusted in consideration of the optimum reaction time.

The reactor portion is a site for regulating the length of the flow path and precisely controlling the time required to carry out the reaction (controlling residence time). In a flow reactor, reaction time is equivalent to the residence time in a flow path of a reaction solution obtained by mixing all raw materials. Since the aforementioned residence time is proportional to flow path length, reaction time is adjusted by adjusting flow path length.

The composition such as the cross-sectional area, inner diameter, outer diameter, flow path length and materials of the flow path of the reactor portion can be suitably selected corresponding to the desired reaction. For example, there are no particular limitations on the material of the reactor portion, and those materials listed as examples of materials of the aforementioned flow reactor can be used preferably.

The mixer portion, introduction paths and reactor portion are provided with connecting means for mutually connecting each member as necessary. There are no particular limitations on the connection method used by the aforementioned connecting means, and can be suitably selected from among known tube connection methods corresponding to the particular purpose, with examples of connection methods including threading, union jointing, butt welding, slip-on welding, socket welding, flange connection, flareless connection, flared connection and mechanical connection.

There are no particular limitations on the members other than the introduction ports, mixer portion and reactor portion, and can be suitably selected corresponding to the particular purpose. Examples of the aforementioned members include pumps used to deliver liquid, temperature control means, reaction accelerating means, sensors, pressure regulating valves, and tanks for storing compounds produced.

There are no particular limitations on the aforementioned pumps, and can be suitably selected from among those able to be used industrially. Among these, pumps that do not generate pulsation during delivery of liquid are preferable, and examples thereof include plunger pumps, gear pumps, rotary pumps and diaphragm pumps.

There are no particular limitations on the aforementioned temperature control means, and can be suitably selected corresponding to the reaction temperature. Examples thereof include constant temperature baths, circulators and heat exchangers.

### [Sodium Azide and Substituent Y Introducing Agent Reaction Conditions]

In the method for producing an azide compound of the present invention, a mixed solvent of water and toluene is used due to the favorable solubility of azide therein. In the case of recovering the azide compound represented by general formula (I) by separating from unreacted sodium azide, a mixed solvent of water and an organic solvent capable of dissolving the azide compound represented by general formula (I) and capable of undergoing phase separation with water is used for the reaction solvent. In this case, the azide compound obtained as a result of synthesis is mainly contained in the organic solvent layer, while the unreacted sodium azide along with salts and other by-products are mainly contained in the aqueous layer. Consequently, the azide compound represented by general formula (I) can be efficiently recovered by recovering the organic solvent layer from the reaction solution following completion of the reaction by separating from the aqueous layer. Toluene is the organic solvent capable of dissolving the azide compound represented by general formula (I).

In addition, in the method for producing an azide compound of the present invention, the reaction is preferably carried out in the presence of base. The presence of base in the reaction solution makes it possible to inhibit the generation of hydrogen azide from the azide. Examples of bases include inorganic bases such as sodium hydroxide, potassium hydroxide, sodium hydride, sodium carbonate or potassium carbonate, and organic bases such as triethylamine, 4-(dimethylamino)pyridine, pyridine, 1,8-diazabicyclo[5.4.0]undecene-7 or 1,5-diazabicyclo[4.3.0]nonene-5. One type of these bases can be used alone or two or more types can be used in combination. Among these, alkaline metal hydroxides such as sodium hydroxide or potassium hydroxide are used preferably.

In addition, the reaction solution may further contain a phase transfer catalyst. A phase transfer catalyst refers to a small amount of reagent used to react an organic compound that is insoluble in water with a reagent that is insoluble in organic solvent. There are no particular limitations on the phase transfer catalyst provided it is inert in the reaction between the azide and substituent Y introducing agent, and can be suitably selected from among known phase transfer catalysts in consideration of such factors as the types and combinations of organic solvents used in the reaction solution. Examples of phase transfer catalysts include quaternary ammonium salts in the manner of tetraalkylammonium chlorides such as tetramethylammonium chloride, tetraethylammonium chloride, tetrapropylammonium chloride or tetrabutylammonium chloride (TBAC), tetraalkylammonium bromides such as tetramethylammonium bromide, tetraethylammonium bromide, tetrapropylammonium bromide or tetrabutylammonium bromide, and benzyltrialkylammonium halides such as benzyltrimethylammonium chloride, benzyltrimethylammonium bromide, benzyltri-n-butylammonium chloride (BTBAC) or benzyltri-n-butylammonium bromide. BTBAC is preferably used as a phase transfer catalyst in the method for producing an azide compound of the present invention.

Although there are no particular limitations on the content ratio of azide and substituent Y introducing agent in the reaction solution, which at least contains both the azide and substituent Y introducing agent and in which an azide compound synthesis reaction occurs in a flow reactor, provided it is a ratio that allows the target reaction to proceed, an adequate amount of substituent Y introducing agent relative to the azide is preferably contained in the aforementioned reaction solution.

In the method for producing an azide compound of the present invention, the reaction solution is prepared by respectively supplying a solution containing the azide and a solution containing the substituent Y introducing agent from separate raw material introduction ports and merging in a mixer portion. From the viewpoint of safety, a method consisting of separately introducing a solution containing the azide and a solution containing the substituent Y introducing agent into the flow reactor is used.

The solution containing the azide and the solution containing the substituent Y introducing agent respectively and independently supplied to the flow reactor are solutions prepared using different types of solvent. For example, the solvent of the solution containing the azide is water or a mixed solvent of water and an organic solvent that does not react with the azide, and is more preferably water. On the other hand, the solvent of the solution containing the substituent Y introducing agent is an organic solvent that is inert in the reaction. In addition, in the case of carrying out the reaction in the presence of base, the base is preferably added to the solution containing the azide. An aqueous solution containing the azide and base and a solution of the substituent Y introducing agent diluted with organic solvent are separately supplied and merged in the mixer portion. In the case of using a phase transfer catalyst in the aforementioned aspect, the phase transfer catalyst may be added to either the aqueous solution containing the azide or the solution containing the substituent Y introducing agent. In addition, the reaction solution may be prepared by preliminarily housing a solution containing a base in the mixer portion, respectively and separately supplying the aqueous azide solution and the solution of the substituent Y introducing agent diluted with organic solvent from raw material introduction ports, and merging in the mixer portion.

The reaction temperature of the reaction solution in the flow reactor (solution following mixing of all raw materials) is a temperature at which risk of decomposition of the synthesized azide compound is adequately inhibited, from the viewpoint of safety, the reaction temperature is preferably a comparatively low temperature. Consequently, the reaction temperature in the method for producing an azide compound of the present invention is 35°C to 80°C.

In the method for producing an azide compound of the present invention, an azide compound represented by the aforementioned general formula (I) is obtained in a flow reactor. The resulting azide compound may be washed with neutral to alkaline water following the reaction in order to remove trace amounts of hydrogen azide or sodium azide present in the solution after the reaction.

### Examples

Although the following provides a more detailed explanation of the present invention through examples thereof, the present invention is not limited to these examples.

### [Example 1]

Methyl azide was synthesized by reacting sodium azide and dimethyl sulfate in a flow reactor.

### <Flow Reactor>

An SUS pipe (2 mm (inner diameter) × 2.8 m (flow path length), residence time during flow rate of 1 mL/min: approximately 9 min) equipped with a back pressure valve and having one end connected to a T-mixer (material: PEEK) and the other end connected to a condenser was immersed in a water bath at 40°C and used as a flow reactor. 50 mL volume glass syringes (syringe A, syringe B) were respectively connected to the remaining two openings of the T-mixer not connected to the SUS pipe, and these glass syringes were respectively attached to syringe pumps.

### <Solution Preparation>

19.56 g (0.30 mol) of sodium azide, 25.77 g of 28% by weight aqueous sodium hydroxide solution (final concentration: 0.72 mol/L) and 9.42 g of 50% by weight aqueous BTBAC solution (final concentration: 0.06 mol/L) were dissolved with 214.4 g of water to prepare a sodium azide solution. The sodium azide concentration of the resulting sodium azide solution was 7.27% by weight (1.2 mol/L).

Separate from the above, 56.83 g of dimethyl sulfate were diluted with 53.72 g of toluene to prepare a dimethyl sulfate solution. The dimethyl sulfate concentration of the resulting dimethyl sulfate solution was 53.17% by weight (4.5 mol/L).

### <Reaction>

35.32 g of the sodium azide solution were filled into syringe A and 24.35 g of the dimethyl sulfate solution were filled into syringe B. Next, the solutions were delivered from each syringe so that the ratio between the liquid volume delivered from syringe A and the liquid volume delivered from syringe B (volume ratio) was 7:3 and the total flow rate was 1 ml/min. As a result, 34.51 g of the sodium azide solution from syringe A (charged amount of sodium azide: 38.46 mmol) and 14.2 g of the dimethyl sulfate solution from syringe B (charged amount of dimethyl sulfate: 59.7 mmol, equal to 1.55 times the number of moles of sodium azide) were introduced into the SUS pipe. Subsequently, a reaction solution obtained by cooling with the condenser was recovered.

The recovered reaction solution was separated into an aqueous layer and an organic solvent layer (toluene layer) and respective amounts of methyl azide and sodium azide contained therein were analyzed by HPLC. The yields (mol%) of methyl azide and sodium azide in each layer based on the charged amount of sodium azide are shown in Table 1. As a result, methyl azide was contained in both the organic solvent layer and aqueous layer and methyl azide was able to be synthesized safely under low temperature conditions of 40°C without requiring a special explosion-proof apparatus. In addition, although the entire amount of unreacted sodium azide was contained in the aqueous layer, nearly 90% of the synthesis product in the form of methyl azide had migrated to the organic solvent layer.

**[Table 1]**

| Yield (mol%) | | |
|---|---|---|
| | MeN₃ | NaN₃ |
| Organic solvent layer | 38.3 | 0.0 |
| Aqueous layer | 5.6 | 28.9 |
| Total | 43.9 | 28.9 |

### [Example 2]

Methyl azide was synthesized by reacting sodium azide and dimethyl sulfate in a flow reactor.

### <Flow Reactor>

An SUS pipe (1 mm (inner diameter) × 19.2 m (flow path length), residence time during flow rate of 1.6 mL/min: approximately 9 min) equipped with a back pressure valve and having one end connected to a T-mixer (material: PEEK) and the other end connected to a condenser was immersed in an oil bath at 80°C and used as a flow reactor. HPLC pumps A and B were respectively connected to the remaining two openings of the T-mixer not connected to the reactor portion with an SUS pipe.

### <Solution Preparation>

3.9g (0.06mol) of sodium azide and 10.29 g of 28% by weight aqueous sodium hydroxide solution (equivalent to 120 mol% based on sodium azide) were weighed into a 50 mL volumetric flask and brought to a final volume of 50 mL with water to prepare a sodium azide solution. The sodium azide concentration of the resulting sodium azide solution was 1.2 mol/L.

Separate from the above, 30.27 g (equivalent to 400 mol%) of dimethyl sulfate were weighed into a 100 mL volumetric flask and brought to a final volume of 100 mL with toluene to prepare a dimethyl sulfate solution. The dimethyl sulfate concentration of the resulting dimethyl sulfate solution was 2.4 mol/L and half that amount was delivered.

### <Reaction>

The sodium azide solution was placed in the aspiration port of the HPLC pump A and the dimethyl sulfate solution was placed in the aspiration port of the HPLC pump B. The HPLC pump A and the HPLC pump B were operated for 60 minutes so that the respective delivery volume was 0.8 mL/min (total flow rate: 1.6 mL/min). As a result, 50 mL of sodium azide solution (charged amount of sodium azide: 0.06 mol) from HPLC pump A and 50 mL of dimethyl sulfate solution (charged amount of dimethyl sulfate: 0.12 mol, equal to 2.0 times the number of moles of sodium azide) from HPLC pump B were introduced into the SUS pipe. Subsequently, pump A was switched to water and pump B was switched to toluene as washing liquids followed by operating for 60 minutes and recovering the reaction solution after cooling with the condenser.

The recovered reaction solution was separated into an aqueous layer and an organic solvent layer (toluene layer) and respective amounts of methyl azide and sodium azide contained therein were analyzed by HPLC. The yields (mol%) of methyl azide and sodium azide in each layer based on the charged amount of sodium azide are shown in Table 2. As a result, methyl azide was contained in both the organic solvent layer and aqueous layer. Yield improved to 74.8 mol% and methyl azide was able to be synthesized safely and at high yield even under temperature conditions of 80°C without requiring a special explosion-proof apparatus. In addition, although the entire amount of unreacted sodium azide was contained in the aqueous layer, nearly 80% of the synthesis product in the form of methyl azide had migrated to the organic solvent layer.

**[Table 2]**

| Yield (mol%) | | |
|---|---|---|
| | MeN₃ | NaN₃ |
| Organic solvent layer | 57.20 | 0.00 |
| Aqueous layer | 17.57 | 10.43 |
| Total | 74.77 | 10.43 |

### INDUSTRIAL APPLICABILITY

The present invention is able to provide a method for efficiently and safely producing an azide compound in a flow reactor by using sodium azide as raw material.

According to the method for producing an azide compound of the present invention, since an azide compound is useful as synthesis raw materials of tetrazoyl oxime derivatives that are useful as active ingredients of agricultural chemicals, pharmaceuticals and the like, can be produced efficiently and safely, the method for producing an azide compound of the present invention can be used in manufacturing fields such as agricultural chemicals or pharmaceuticals.

## Claims

1. A method for producing an azide compound, comprising:
reacting an alkylating agent with sodium azide in a flow reactor to produce an azide compound represented by the following general formula (I):
**Y-N₃ ···** **(I)**
wherein, Y represents a linear alkyl group having 1 to 2 carbon atoms in the state of a solution; and
recovering the azide compound represented by the formula (I) in the state of a solution,
wherein the reaction is carried out at a reaction temperature ranging from 35°C to 80°C, and the boiling point of the azide compound produced is lower than the reaction temperature,
wherein a solution containing the alkylating agent and a solution containing the sodium azide are respectively and separately introduced into the flow reactor, and
the solvent of the solution containing the sodium azide contains water, and the solvent of the solution containing the alkylating agent is an organic solvent capable of dissolving the azide compound represented by the general formula (I) and capable of undergoing phase separation with water, wherein the organic solvent is toluene, and the alkylating agent is a compound represented by formula (III-B): 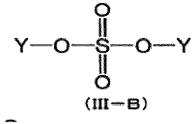 wherein, Y is the same as defined in the general formula (I) .

2. The method for producing an azide compound according to claim 1, wherein the solution containing the sodium azide further contains a base.

## Patentansprüche

1. Verfahren zur Herstellung einer Azid-Verbindung, das Folgendes umfasst:
das Umsetzen eines Alkylierungsmittels mit Natriumazid in einem Strömungsreaktor, um eine Azid-Verbindung der folgenden allgemeinen Formel (I):
**Y-N₃ ...** **(I)**
worin Y für eine unverzweigte Alkylgruppe mit 1 oder 2 Kohlenstoffatomen steht,
in Form einer Lösung herzustellen; und
das Gewinnen der Azid-Verbindung der Formel (I) in Form einer Lösung;
wobei die Reaktion bei einer Reaktionstemperatur von 35 °C bis 80 °C durchgeführt wird und der Siedepunkt der hergestellten Azid-Verbindung unter der Reaktionstemperatur liegt;
wobei eine das Alkylierungsmittel enthaltende Lösung und eine das Natriumazid enthaltende Lösung jeweils und getrennt voneinander in den Strömungsreaktor eingeleitet werden und
wobei das Lösungsmittel der das Natriumazid enthaltenden Lösung Wasser enthält und das Lösungsmittel der das Alkylierungsmittel enthaltenden Lösung ein organisches Lösungsmittel ist, das in der Lage ist, die Azid-Verbindung der allgemeinen Formel (I) zu lösen und in der Lage ist, eine Phasentrennung mit Wasser zu erfahren, wobei das organische Lösungsmittel Toluol ist;
und wobei das Alkylierungsmittel eine Verbindung der Formel (III-B) ist: 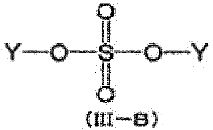 worin Y gleich wie in der allgemeinen Formel (I) definiert ist.

2. Verfahren zur Herstellung einer Azid-Verbindung nach Anspruch 1, wobei die das Natriumazid enthaltende Lösung außerdem eine Base enthält.

## Revendications

1. Procédé de production d'un composé azide, comprenant les étapes consistant à :
faire réagir un agent alkylant avec de l'azide de sodium dans un réacteur à écoulement pour produire un composé azide représenté par la formule générale (I) suivante :
Y-N₃ ··· (I)
dans lequel, Y représente un groupe alkyle linéaire ayant 1 à 2 atomes de carbone à l'état d'une solution ; et
récupérer le composé azide représenté par la formule (I) à l'état de solution,
dans lequel la réaction est mise en œuvre à une température de réaction dans la plage de 35°C à 80°C, et le point d'ébullition du composé azide produit est inférieur à la température de réaction,
dans lequel une solution contenant l'agent d'alkylation et une solution contenant l'azide de sodium sont respectivement et séparément introduites dans le réacteur à écoulement, et
le solvant de la solution contenant l'azide de sodium contient de l'eau, et le solvant de la solution contenant l'agent d'alkylation est un solvant organique capable de dissoudre le composé azide représenté par la formule générale (I) et capable de subir une séparation de phase avec de l'eau, dans lequel le solvant organique est le toluène,
et l'agent d'alkylation est un composé représenté par la formule (III-B) : 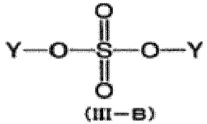 dans lequel, Y est le même que celui défini dans la formule générale (I).

2. Procédé de production d'un composé azide selon la revendication 1, dans lequel la solution contenant l'azide de sodium contient en outre une base.
